(19) Europäisches Patentamt
European Patent Office
Office européen des brevets

(11) Publication number: **0 149 229**
**B1**

(12) **EUROPEAN PATENT SPECIFICATION**

(45) Date of publication of patent specification: **14.06.89**

(51) Int. Cl.$^4$: **C 07 D 261/20**

(21) Application number: **84116202.7**

(22) Date of filing: **23.04.82**

(60) Publication number of the earlier application in accordance with Art. 76 EPC: **0 063 799**

(54) **3-(Gamma-chloropropyl)-6-fluor-1,2-benzisoxazole, process for its preparation and its use as an intermediate.**

(30) Priority: **27.04.81 US 257698**
**09.04.82 US 366247**

(43) Date of publication of application:
**24.07.85 Bulletin 85/30**

(45) Publication of the grant of the patent:
**14.06.89 Bulletin 89/24**

(84) Designated Contracting States:
**AT BE CH DE FR GB IT LI LU NL SE**

(56) References cited:
**CHEMICAL ABSTRACTS, vol. 87, no. 5, 1st August 1977, Columbus, OH (US); p. 519, no. 39459c**

(73) Proprietor: **HOECHST-ROUSSEL PHARMACEUTICALS INCORPORATED**
**Route 202-206 North**
**Somerville New Jersey 08876 (US)**

(72) Inventor: **Davis, Larry**
**P.O.Box 129**
**Sergeantsville, NJ 08557 (US)**
Inventor: **Effland, Richard Charles**
**544 Rolling Hills Road**
**Bridgewater, NJ 08807 (US).**
Inventor: **Klein, Joseph Thomas**
**P.O.Box 6576**
**Bridgewater, NJ 08807 (US)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 149 229 B1**

Courier Press, Leamington Spa, England.

**Description**

The present invention relates to 3-(γ-chloropropyl)-6-fluoro-1,2-benzisoxazole, a process for the preparation thereof and its use in the synthesis of 1-[3-(6-fluoro-1,2-benzisoxazol-3-yl)propyl]-4-phenyl-4-hydroxypiperidines which are useful as neuroleptics. These neuroleptics are subject of the EP—A—63799.

The benzisoxazole having the structural formula

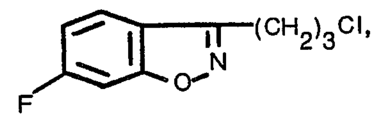

is prepared by cyclizing an oxime derivative having the formula

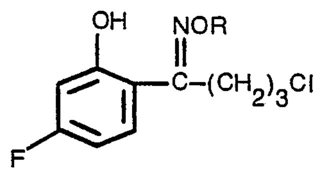

where R is a substituent selected from

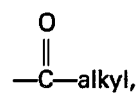

where alkyl is a straight or branched chain hydrocarbon of 1 to 5 carbon atoms, and

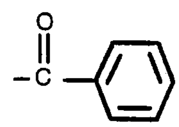 ,

Japanese patent publication JP—A—76/136—666 discloses a broad class of compounds of the formula I as starting materials for drugs exhibiting action on the central nervous system,

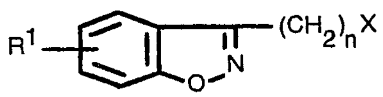 I

where $R_1$ is hydrogen, halogen atom, lower alkoxy, or hydroxy; X is a reactive ester residue of an alcohol (e.g., chlorine, bromine, iodine, an aryl sulfonyloxy group, such as p-toluene-sulfonyloxy or benzene sulfonyloxy, or an alkyl sulfonyloxy group, such as methanesulfonyloxy; and n is an integer 1—3.

This disclosure, however, does not reveal or hint that 3-(-chloropropyl)-6-fluoro-1,2-benzisoxazole is especially useful in the synthesis of neuroleptics, nor does it reveal or hint at the preparation of this precursor or a method by which to do so.

Japanese patent publication JP—A—74/070 963 describes the preparation of 1,2-benzisoxazole derivatives of formula II,

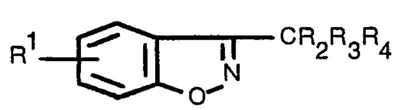 II

where

$R_1$ is hydrogen, hydroxy, alkoxy, alkyl, halogen, nitro;

$R_2$ is halogen; $R_3$ is hydrogen, halogen; $R_4$ is hydrogen, halogen, carboxyl, alkoxycarbonyl by halogenating 1,2-benzisoxazole-3-acetic acid derivatives III,

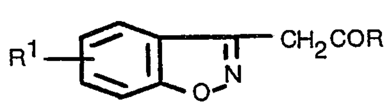 III

where R is hydroxy, alkoxy, halogen; and $R_1$ is as shown under II.

No method, however, is revealed or hinted at which would yield 3-(γ-chloropropyl)-6-fluoro-1,2-benzisoxazole of this invention or even closely related analogs or next adjacent homologs.

A process for preparation of a 3-(γ-substituted propyl)-6-fluoro-1,2-benzisoxazole is described. The process comprises cyclizing an oxime having the formula

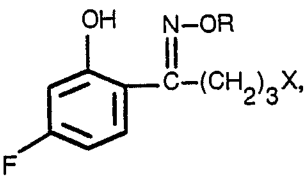

where X is a leaving group in organic nucleophilic displacement reactions. Typical leaving groups are a halide group, comprising Cl and Br, a tosyl group, a mesyl group, etc. A preferred halide is Cl. R is a substituent selected from

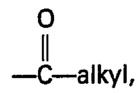

where alkyl is a straight or branched chain hydrocarbon of 1 to 6 carbon atoms, and

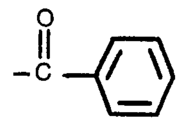 ,

The starting compound for the preparation of 3-(γ-substituted-propyl)-6-fluoro-1,2-benzisoxazole is γ-substituted-4-fluoro-2-hydroxybutyrophenone. A preferred compound is γ-chloro-4-fluoro-2-hydroxybutyrophenone having a formula,

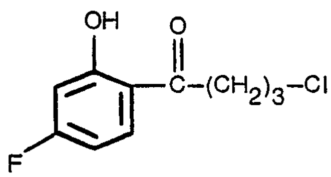

A butyrophenone precursor to this benzisoxazole is prepared by reacting 3-fluorophenol, with an acid chloride, such as the acid chloride

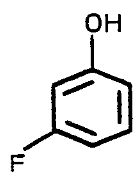 , 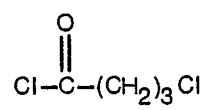

in the manner described in Belgian patent 839,097.

The resultant butyrophenone, e.g. γ-chloro-4-fluoro-2-hydroxybutyrophenone, is then converted into its oxime, e.g.

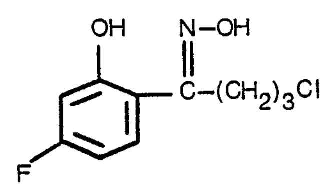

by reaction with a salt of hydroxylamine, utilizing conventional techniques and conditions. Typically hydroxylamine hydrochloride is employed in the presence of an acid acceptor, such as pyridine.

The resulting oxime, γ-chloro-4-fluoro-2-hydroxybutyrophenone oxime, is predominantly the E-oxime in respect to its position relative to the benzene ring. This reasoning is deduced from the fact that after acetylation to E-γ-chloro-4-fluoro-2-hydroxybutyrophenone-O-acetyl oxime, the acetylated oxime gives a high yield (80%) of 3-(γ-chloropropyl)-6-fluoro-1,2-benzisoxazole on cyclization.

The resulting oxime, e.g.

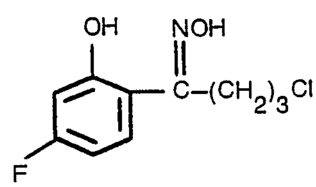

is reacted with an acyle anhydride, acetyl halide, e.g., chloride, or benzoic anhydride or halide to form an O-acetyl or O-benzoyl compound e.g.

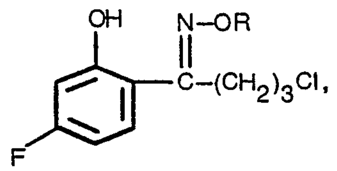

where R is selected from

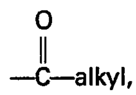

where alkyl is a straight or branched chain hydrocarbon substituent having 1 to 6 carbon atoms, and

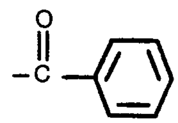

,

It is to be understood that other reagents reacting with the —OH group of the oxime may be employed to form O-alkanoyl or O-aroyl derivatives, as for example, propionic anhydride, butyric anhydride, acetic benzoic anhydride, propionyl chloride, butyroyl chloride, and benzoyl chloride.

The resulting O-alkanoyl or O-aroyl derivative is then cyclized in the presence of a base to form the desired compound

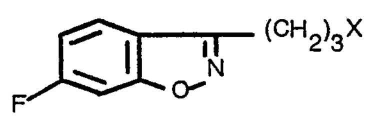

e.g.,

3-(γ-chloropropyl)-6-fluoro-1,2-benzisoxazole. Typically the cyclization is carried out in the presence of potassium carbonate.

Alternate reagents of potassium carbonate to effect cyclization of the O-acyl oxime, e.g., E-γ-chloro-4-fluoro-2-hydroxybutyrophenone-O-acetyl oxime, include alkaline reagents such as sodium bicarbonate, sodium carbonate, calcium carbonate, magnesium carbonate, calcium oxide, magnesium oxide and potassium hydroxide. Because of the necessity to avoid excess alkali with its potential for reaction with the chlorine atom of the chloropropyl side chain attached to the benzisoxazole ring, it is desirable to limit any alkali present to a slight excess over that required to react with the phenol group. Desirably, alkali remaining after reaction with the acetylated oxime should not cause a pH greater than 9 after dilution with water. Cyclization with reagents yielding a pH after reaction and dilution with water below 8 are preferred.

It is understood that the above-identified reactions of the reaction sequence are carried out in a conventional solvent system typically employed therefor and well known to those skilled in the art.

The overall reaction sequence from γ-chloro-4-fluoro-2-hydroxy-butyrophenone to its oxime to its acetylated oxime to 3-(γ-chlorpropyl)-6-fluoro-1,2-benzisoxazole is remarkable for its high yield of desired product. This yield is the more remarkable in that the γ-chloropropyl group suvives intact and is available for subsequent use in reactions to prepare compounds having neuroleptic utility, such as for example 4-(4-chlorophenyl)-1-[3-(6-fluoro-1,2-benzisoxazole-3-yl)propyl]-4-hydroxypiperidine.

By condensing 3-(γ-chloropropyl)-6-fluoro-1,2-benzisoxazole with readily available 4-hydroxy-4-phenyl-piperidines of the formula

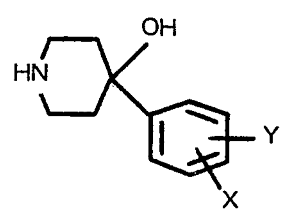

in the presence of an acid acceptor, a displacement promotor and a suitable solvent of the formula

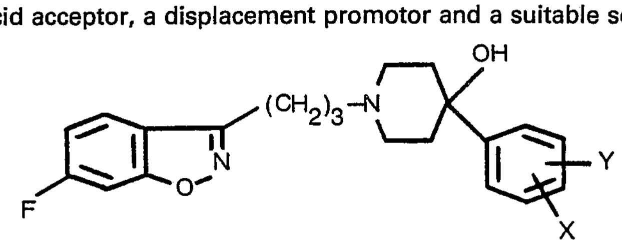

are obtained which are useful in neuroleptics.

Example 1

γ-Chloro-4-fluoro-2-hydroxybutyrophenone

To 350 ml (2.8 mols) cold boron trifluoride etherate is added 100 g (0.89 mole) of 3-fluorophenol and 252 g (200 ml 1.78 mole) of 4-chlorobutyryl chloride. The resulting solution is stirred for twenty hours at 130°C.

The mixture is cooled and poured into one liter of mixed ice and water. After stirring for ten minutes, the water mixture is extracted three times with 200 ml of ether each time. The ether extracts are combined and washed three times with water. The ether extract is dried by washing with a saturated solution of sodium chloride in water and is then dried over anhydrous magnesium sulfate.

The magnesium sulfate is filtered from the dry ether solution and the ether is evaporated. The resultant oil is vacuum distilled and a fraction is collected at 110—150°C (1.0 mm, pot at 170—250°C) weighing 115 g (50% of the theoretical yield). This material is recrystallized from a mixed solvent solution (hexane/ether at 8:1 ratio) to obtain white crystals of γ-chloro-4-fluoro-2-hydroxybutyrophenone, melting at 68—70°C.

Example 2

E-γ-chloro-4-fluoro-2-hydroxybutyrophenone oxime

To 25 ml of pyridine is added 11.2 g (0.52 mole) of γ-chloro-4-fluoro-2-hydroxybutyrophenone of Example 12 and 4.14 g (0.06 mole) of hydroxylamine hydrochloride. This mixture is stirred overnight (20 hours) at ambient temperature. It is then poured into 100 ml of dilute hydrochloric acid, stirred for five minutes, and is extracted with ether. The ether solution is washed two times with water before it is dried by washing with a saturated solution or sodium chloride in water followed by final drying over anhydrous magnesium sulfate.

The magnesium sulfate is filtered from the ether solution, and the ether is evaporated. The white solid obtained is recrystallized from a mixed hexane and ether solvent to obtain 11.5 g (95% of the theoretical yield) of white crystals melting at 68—70°C. A sample of this material is again recrystallized from hexane/ether to yield white crystals of E-γ-chloro-4-fluoro-2-hydroxybutyrophenone oxime, melting at 74—6°C.

| Analysis: | | | |
|---|---|---|---|
| Calculated for $C_{10}H_{11}ClFNO_2$: | 51.84%C | 4.79%H | 6.05%N |
| Found: | 52.20%C | 4.77%H | 6.00%N |

Example 3

E-γ-chloro-4-fluoro-2-hydroxybutyrophenone-O-acetyl oxime

To 5.5 ml (0.06 mole) of acetic anhydride is added 10.0 g (0.043 mole) of E-γ-chloro-4-fluoro-2-hydroxybutyrophenone oxime.

This mixture is heated at 60°C for one and one-half hours. The resulting mixture is cooled to room temperature and is dissolved in 100 ml of ether. The ether solution is washed twice with a sodium bicarbonate solution in water. It is then washed twice with water. The ether solution remaining is dried by washing with a saturated solution of sodium chloride in water before finally drying it over anhydrous magnesium sulfate.

The magnesium sulfate is removed by filtration after drying is complete and the ether is evaporated from the dry ether solution to give a solid which is recrystallized from a mixed hexane/ether solvent to obtain 9.0 g (76% of the theoretical yield) of E-γ-chloro-4-fluoro-2-hydroxybutyrophenone-O-acetyloxime possessing a melting point of 84—87°C. A sample of this material is recrystallized from a mixed hexane/ether solvent. The crystals of E-γ-chloro-4-fluoro-2-hydroxy-butyrophenone-O-acetyloxime which are obtained melt at 86—88°C.

| Analysis: | | | |
|---|---|---|---|
| Calculated for $C_{12}H_{13}ClFNO_3$: | 52.66%C | 4.79%H | 5.22%N |
| Found: | 52.56%C | 4.81%H | 5.05.%N |

Example 4

3-[γ-chloropropyl]-6-fluoro-1,2-benzisoxazole

The cyclization of the acetylated oxime of Example 3 is effected by adding 6.5 g (0.024 mole) of E-γ-chloro-4-fluoro-2-hydroxy-butyrophenone-O-acetyl oxime to 20 ml of dry dimethylformamide (DMF) and 4.14 g (0.03 mole) of potassium carbonate. This mixture is stirred for five hours at room temperature. It is poured into 500 ml of water, stirred for five minutes, and is then extracted with a mixed solvent of ether/ethyl acetate. The extracted organic layer is washed two times with water and once with a saturated solution of sodium chloride in water. The organic layer is finally dried over anhydrous magnesium sulfate.

After drying, the magnesium sulfate is removed by filtration and the solution is evaporated to remove ether and ethyl acetate. The product is an oil. This oil is distilled to yield 4.0 g (80% of the theoretical yield) of an oil of 3-(γ-chloropropyl)-6-fluoro-1,2-benzisoxazole boiling at 130°C/0.5 mm.

| Analysis: | | | |
|---|---|---|---|
| Calculated for $C_{10}H_9ClFNO$: | 56.22%C | 4.25%H | 6.56%N |
| Found: | 56.21%C | 4.25%H | 6.52%N |

Example 5

4-(4-Chlorophenyl-1-[3-(6-fluoro-1,2-benzisoxazole-3-yl)-propyl]-4-hydroxypiperidine

In 30 ml dry DMF is added 4-(4-chlorophenyl)-4-hydroxypiperidine (3.0 g, 0.014 mole) 3-(γ-chlorophenyl)-6-fluoro-1,2-benzisoxazole (2.99 g, 0.014 mole), $NaHCO_3$ (8.0 g, 0.1 mole), and 0.01 g of KI. After stirring at 90°C for one hour, the mixture is evaporated to an oil, which is stirred with 100 ml water for five minutes and then extracted with ether. The ether solution is washed twice with water and then dried (saturated NaCl, anhydrous $MgSO_4$). After filtering, the solvent is evaporated to yield 4.7 g of solid, m.p. 130°C. The solid material is recrystallized from ether to yield 3,5 g (64%) m.p. 143—5°C. This material is recrystallized from ether to yield 3.0 g of a solid of 4-(4-chlorophenyl)-1-[3-(6-fluoro-1,2-benzisoxazole-3-yl)propyl]-4-hydroxypiperidine, m.p. 148—50°C.

| | | | |
|---|---|---|---|
| Analysis: | | | |
| Calculated for $C_{21}H_{22}ClFN_2O_2$: | 64.85%C | 5.70%H | 7.21%N |
| Found: | 64.75%C | 5.64%H | 7.15%N |

**Claims for the Contracting States: BE CH DE FR GB IT LI LU NL SE:**

1. The compound 3-[γ-chloropropyl)-6-fluoro-1,2-benzisoxazole.

2. A process for preparing 3-(γ-chloropropyl)-6-fluoro-1,2-benzisoxazole wihich comprises cyclizing an oxime derivative having the formula of

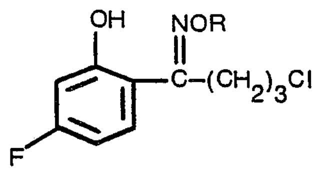

where R is a substituent selected from the group consisting of

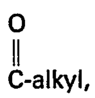

where alkyl is a straight or branched chain hydrocarbon substituent of 1 to 6 carbon atoms, and

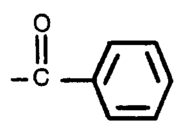 ,

3. The process as defined in claim 2 wherein said cyclization is carried out in the presence of an alkaline reagent.

**Claims for the Contracting State: AT**

1. A process for preparing 3-(γ-chloropropyl)-6-fluoro-1,2-benzisoxazole which comprises cyclizing an oxime derivative having the formula of

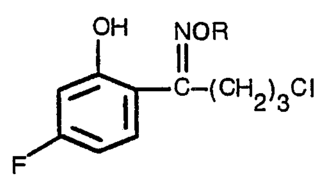

where R is a substituent selected from the group consisting of

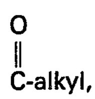

where alkyl is a straight or branched chain hydrocarbon substituent of 1 to 6 carbon atoms, and

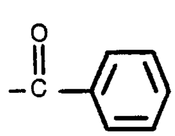 ,

2. The process as defined in claim 1 wherein said cyclization is carried out in the presence of an alkaline reagent.

**Patentansprüche für die Vertragsstaaten: BE CH DE FR GB IT LI LU NL SE**

1. Die Verbindung 3-(γ-Chloropropyl)-6-fluor-1,2-benzisoxazol.

2. Ein Verfahren zur Herstellung von 3-(γ-Chloropropyl)-6-fluor-1,2-benzisoxazol, gekennzeichnet durch das Cyclisieren eines Oximderivats mit der Formel

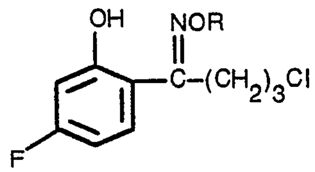

wobei R aus der aus

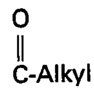

bestehenden Gruppe, wobei Alkyl ein unverzweigter oder ein verzweigter Kohlenwasserstoffsubstituent von 1 bis 6 Kohlenstoffatomen ist, und

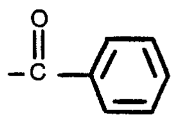 ,

ausgewählt ist:

3. Das wie in Anspruch 2 definierte Verfahren, worin die genannte Cyclisierung in Anwesenheit eines alkalischen Reagens ausgeführt wird.

**Patentansprüche für den Vertragsstaat AT:**

1. Ein Verfahren zur Herstellung von 3-(γ-Chloropropyl)-6-fluor-1,2-benzisoxazol, gekennzeichnet durch das Cyclisieren eines Oximderivats mit der Formel

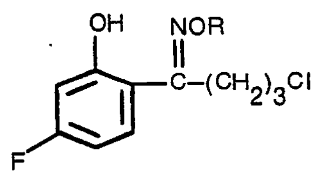

wobei R aus der aus

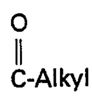

bestehenden Gruppe, wobei Alkyl ein unverzweigter oder ein verzweigter Kohlenwasserstoffsubstituent von 1 bis 6 Kohlenstoffatomen ist, und

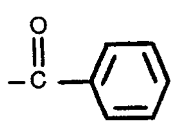 ,

ausgewählt ist:

2. Das wie in Anspruch 1 definierte Verfahren, worin die genannte Cyclisierung in Anwesenheit eines alkalischen Reagens ausgeführt wird.

**Revendications pour les Etas contractants: BE CH DE FR GB IT LI LU NL SE**

1. Le composé 3-(γ-chloropropyl)-6-fluoro-1,2-benzisoxazole.

2. Procédé de préparation du 3-(γ-chloropropyl)-6-fluoro-1,2-benzisoxazole qui comprend la cyclisation d'un dérivé d'oxime ayant la formule

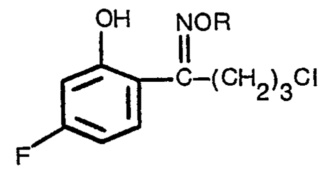

dans laquelle R est un substituant choisi parmi

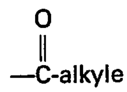

où "alkyle" est un substituant hydrocarboné à chaîne droite ou ramifiée ayant 1 à 6 atomes de carbone, et

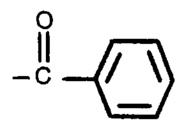 ,

3. Procédé selon la revendication 2 dans lequel la cyclisation est réalisée en présence d'un réactif alcalin.

**Revendications pour l'Etat contractant AT:**

1. Procédé de préparation du 3-(γ-chloropropyl)-6-fluoro-1,2-benzisoxazole qui comprend la cyclisation d'un dérivé d'oxime ayant la formule

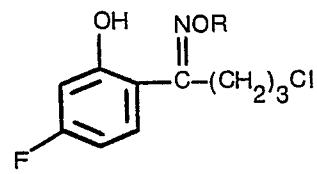

dans laquelle R est un substituant choisi parmi

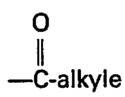

où "alkyle" est un substituant hydrocarboné à chaîne droite ou ramifiée ayant 1 à 6 atomes de carbone, et

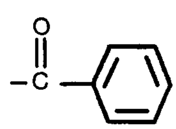 ,

2. Procédé selon la revendication 2 dans lequel la cyclisation est réalisée en présence d'un réactif alcalin.